# EUROPÄISCHE PATENTSCHRIFT

(11) **EP 1 151 005 B1**
(45) Veröffentlichungstag und Bekanntmachung des Hinweises auf die Patenterteilung: **18.06.2003**
(21) Anmeldenummer: 00916753.7
(22) Anmeldetag: 17.02.2000
(51) Int. Cl.: C07K 14/705

(54) **PEPTID-VARIANTEN DES TUMORMARKERS MUC1 UND IHRE VERWENDUNG**
PEPTIDE VARIANTS OF THE TUMOUR MARKER MUC1 AND THE USE THEREOF
VARIANTES PEPTIDIQUES DU MARQUEUR TUMORAL MUC1 ET LEUR UTILISATION

(30) Priorität: 17.02.1999 DE 19906615
(43) Veröffentlichungstag der Anmeldung: 07.11.2001
(73) Patentinhaber: Cell Center Cologne GmbH, 50931 Köln (DE)
(72) Erfinder: HANISCH, Franz-Georg, D-50679 Köln (DE); MÜLLER, Stefan, D-51465 Bergisch-Gladbach (DE)
(74) Vertreter: Baumbach, Friedrich, Dr.
(86) Internationale Anmeldenummer: DE0000440
(87) Internationale Veröffentlichungsnummer: WO00049045

(56) Entgegenhaltungen:
- WO-A-94/22464
- WO-A-98/37095
- PATENT ABSTRACTS OF JAPAN vol. 1995, no. 05, 30. Juni 1995 (1995-06-30) & JP 07 051065 A (NIPPON KOUTAI KENKYUSHO:KK;OTHERS: 01), 28. Februar 1995 (1995-02-28)

## Beschreibung

Die Erfindung betrifft Peptid-Varianten des Tumormarkers MUC1 und ihre Verwendung in antigenen und immunogenen Mitteln. Sie betrifft konkret Peptide der MUC1-Wiederholungseinheit innerhalb der VNTR (variable number of tandem repeats)-Domäne.

Epitheliale Mucine sind Glykoproteine mit repetitiven Aminosäuresequenzen und einem hohen Kohlenhydratanteil, die teils membrangebunden sind, teils sezerniert werden und auf vielen Drüsenepithelien vorkommen. Das am besten bekannte epitheliale Mucin ist das membrangebundene MUC1, auch als PEM, PUM, EMA, MAM-6, PAS-O oder Episialin beschrieben (Finn, O., et al., Immunol.Reviews 145:61, 1995).

Das MUC1 ist an sich kein tumorspezifisches Molekül; seine Eignung als Tumorantigen beruht darauf, daß sein Kohlenhydratanteil bei Tumoren qualitativ und quantitativ verändert ist (Burchell,J., und Taylor-Papadimitriou, J., Epith.Cell Biol. 2:155, 1993). Dabei treten neue Epitope auf, die vom Immunsystem (humorale und zelluläre Abwehr) wahrgenommen werden.

Der extrazelluläre Teil von MUC1 besteht aus einer variablen Anzahl sich wiederholender Einheiten aus 20 Aminosäuren, den sogenannten "Tandem-Repeats". Diese MUC1-Wiederholungseinheit der VNRT (variable number of tandem repeats)-Domäne wurde von Gendler, S. et al., 1990, J. Biol. Chem. 265, 15286-15293 auf der DNA-Basis als Icosapeptid mit der Sequenz PAPGSTAPPAHGVTSAPDTR (PAP20) beschrieben und unter den AC-Nummern J03651, J05288 und J05581 bei der GenBank™/EMBL registriert (siehe auch die unter den Genbank-Eintragungen angegebenen Literaturstellen). Diese Sequenz wird beim Menschen als streng konserviert angesehen, da bisher keine Strukturvarianten gefunden wurden.

In JP-A-7051065 wird ein als Glykoprotein 39 bezeichnetes mucin-artiges Protein offenbart, wobei die aufgeführte DNA- bzw. Proteinsequenz einem c-terminalen Abschnitt der VNTR-Domäne und den transmembranen und cytosolischen Domänen des MUC1 entspricht.

In WO-A-9837095 wird ein rekombinantes POX Virus zur Immunisierung degen MUC1 Tumor-assoziiertes Antigen beschrieben. Es wird eine bestimmte Peptidvariante des MUC1 innerhalb der VNTR-Domäne beschrieben (Variation in Position 19) ohne jedoch ein mögliches tumordiagnostisches oder therapeutisches Potential anzugeben.

Der Erfindung lag die Aufgabe zugrunde, Peptid-Varianten zu suchen, die zur Generierung spezifischer Immundiagnostika geeignet sind.

Überraschend ergaben sich neue Strukturerkenntnisse aus Sequenzierungsanalysen auf Protein- und DNA-Ebene, die für sezerniertes MUC1 normaler Brustdrüsenzellen (Milch) und verschiedener Karzinomzellen gewonnen wurden.

Die Erfindung beruht auf dem Befund, daß das aus Milch isolierte MUC1 keine Varianten der bekannten PAP20-Sequenz aufweist, während menschliche Karzinomzellen einen hohen Anteil alternativer Sequenzen der VNTR-Peptide zeigen.

Erfindungsgemäß wurden in den Sequenzen des PAP20-Peptids 3 Aminosäureaustausche festgestellt:
Pro9 → Ala, Asp18 → Glu, Thr19 → Ser,
die durch Massenspektrometrie und quantitativen Edman-Abbau identifiziert wurden (siehe Ausführungsbeispiel).

Gegenstand der Erfindung sind somit Peptid-Varianten des MUC1 innerhalb der VNTR (variable number of tandem repeats)-Domäne umfassend 20 Aminosäuren, die an den Positionen 9, 18 und 19 oder an den Positionen 18 und 19 der PAP20-Sequenz von der bekannten VNTR-Peptidsequenz abweichen.

Bevorzugt sind es die Peptide mit der SEQ ID No. 1: PAPGSTAPAAHGVTSAPDTR (PAP20-A), mit der SEQ ID No. 2: PAPGSTAPPAHGVTSAPESR (PAP20-ES), und mit der SEQ ID No. 3:

PAPGSTAPAAHGVTSAPESR (PAP20-AES)

Die Pro → Ala Substitution hat starken Einfluß auf die Sekundärstruktur des Peptids, da Prolinreste wesentlich an der Ausbildung einer linksgewundenen Poly-L-Prolin II-Helix beteiligt sind. Durch die mit Pro → Ala Substitution in Position 9 gegebenen strukturellen Änderungen ergeben sich Einflüsse auf die Antigenität des VNTR-Peptids. Dies gilt in verstärktem Maße für das PDTR-Motiv innerhalb der Wiederholungseinheit, das als intern stabilisiertes Strukturelement mit den Charakteristika einer 'Ausstülpung' den immundominanten Target des VNTR-Peptids darstellt. Der (konservative) Austausch zweier Aminosäuren des Motivs (Asp18-Thr19 gegen Glu18-Ser19) beeinflußt aufgrund der veränderten Kettenlängen der Aminosäurereste sowohl die Antigenität als auch die Immunogenetät des Peptids. Da die spezifischen Aminosäureaustausche innerhalb der VNTR-Domäne bislang nur auf Tumor-MUC1 gefunden wurden, besitzen die veränderten Peptidepitope ein hohes immundiagnostisches und tumortherapeutisches Potential.

Die Peptide können nach bekannten Verfahren durch FestphasenSynthese oder gentechnisch hergestellt werden.

Eine Verwendung der erfindungsgemäßen Peptide besteht in immunogenen Mitteln, im Sinne von spezifischen Immundiagnostika oder in entsprechenden Tumorvakzinen. Diese enthalten mindestens eines der erfindungsgemäßen Peptide.

Die effektivste adjuvante Immuntherapie ist die Vakzinierung. Zwei Voraussetzungen sind hierfür erforderlich: erstens, daß ein geeignetes Zielantigen (Epitop) auf den Tumorzellen vorhanden ist, und zweitens, daß es gelingt, eine möglichst stark immunogene, vorzugsweise synthetische Form einer Vakzine herzustellen.

Es werden erfindungsgemäß Tumorvakzine auf der Grundlage der Molekülstruktur des menschlichen epithelialen Mucins MUC1 bereitgestellt, die vorzugsweise zur Bekämpfung der nach chirurgischer Behandlung oder nach einer anderen primären Therapie noch im Körper verbliebenen Tumorzellen ('minimal residual disease') dienen und die die vom MUC1 abgeleiteten Peptid-Varianten der MUC1-VNTR-Domäne, vorzugsweise Peptid SEQ ID No. 1, 2 und/oder 3 enthalten.

Eine solche Tumorvakzine kann zur Bekämpfung von Tumorzellen vorzugsweise aus Mamma-, colorectalen oder Pankreaskarzinomen im Sinne einer aktiven spezifischen Immunisierung eingesetzt werden.

Weiterhin betrifft die Erfindung analytische Verfahren zur Bestimmung der Identität und Inzidenz von DNA-Mutationen, die den oben spezifizierten Peptidvarianten zugrundeliegen.

Durch Entwicklung eines Testkits auf der Basis dieser DNA-Mutationen lassen sich prognostische Parameter für die Tumordiagnostik erstellen. Das Testprinzip stellt ein PCR-ELISA (= polymerase chain reaction - enzyme immunoassay) auf der Grundlage amplifizierter VNTR-Domänen aus genomischer DNA dar.

Dieser Nachweis dient erfindungsgemäß als Grundlage für Therapieverfahren.

### Ausführungsbeispiel

Am Beispiel der menschlichen Brustkarzinomzellinie T47D soll erläutert werden, welche Evidenz für das Vorliegen alternativer Sequenzen des VNTR-Peptids erbracht werden konnte.

Die sezernierte Glykoform des MUC1 wurde aus Kulturüberständen der Zellinie durch AffinitätsChromatographie auf immobilisiertem Anti-MUC1-Antikörper (BC-3) gereinigt. Nach partieller enzymatischer Deglykosylierung (α-Sialidase, β-Galactosidase) wurde die VNTR-Domäne des Mucins durch die Arg-C spezifische Endoproteinase Clostripain in Icosapeptide (PAP20) fragmentiert. Die durch rpHPLC gereinigten Glykopeptide wurden massenspektrometrisch (QTOF-ESI-Massenspektrometrie) und durch quantitativen Edman-Abbau sequenziert. Die kombinierten Meßdaten lassen sich zweifelsfrei in der Weise interpretieren, daß
(1) alle fünf Positionen des VNTR-Peptids glykolysiert vorliegen, und
(2) neben der bekannten PAP20-Sequenz drei alternative Icosapeptide mit einer Gesamtinzidenz von >50% existieren.

Während der nicht-konservative Pro9 → Ala Austausch auch unabhängig auftritt und insgesamt in etwa 30% der VNTR-Peptide vorliegt, erfolgen der Asp18 → Glu und der Thr19 → Ser Austausch konzertiert in etwa 50% der VNTR-Peptide. Eine vergleichbar hohe Inzidenz dieser Aminosäure-Austausche wurde auf DNA-Ebene für eine Reihe anderer menschlicher Karzinomzellen gefunden. Neben dem bekannten Polymorphismus des MUC1 hinsichtlich der Zahl der Wiederholungseinheiten deutet sich damit ein weiterer genetischer Polymorphismus auf der Ebene der Peptidsequenzen an.

## Patentansprüche

1. Peptid-Varianten des MUC1 innerhalb der VNTR (variable number of tandem repeats)-Domäne umfassend 20 Aminosäuren, die an den Positionen 9, 18 und 19 oder an den Positionen 18 und 19 der PAP20-Sequenz von der bekannten VNTR (variable number of tandem repeats)-Domäne abweichen.

2. Peptid-Varianten nach Anspruch 1, **dadurch gekennzeichnet, daß** sie die SEQ ID Nr. 2 PAPGSTAPPAHGVTSAPESR (PAP20-ES) aufweist.

3. Peptid-Varianten nach Anspruch 1, **dadurch gekennzeichnet, daß** sie die SEQ ID Nr. 3 PAPGSTAPAAHGVTSAPESR (PAP20-AES) aufweist.

4. Immunogenes Mittel, **dadurch gekennzeichnet, daß** es mindestens ein Peptid gemäß der Ansprüche 1 bis 3 oder das Peptid SEQ ID Nr. 1 PAPGSTAPAAHGVTSAPDTR (PAP20-A) enthält.

5. Antigenes Mittel, **dadurch gekennzeichnet, daß** es mindestens ein Peptid gemäß der Ansprüche 1 bis 3 oder das Peptid SEQ ID Nr. 1 PAPGSTAPAAHGVTSAPDTR (PAP20-A) enthält.

6. Testkit zum Nachweis der Identität und Inzidenz von DNA-Mutationen innerhalb der VNTR-Domäne des MUC1 enthaltend mindestens einen DNA-Primer, dessen Sequenz einem der Peptide gemäß Anspruch 1 bis 3 oder dem Peptid SEQ ID Nr. 1 PAPGSTAPAAHGVTSAPDTR (PAP20-A) entspricht.

7. Verwendung der Peptide gemäß Anspruch 1 bis 3 oder des Peptids SEQ ID Nr. 1 PAPGSTAPAAHGVTSAPDTR (PAP20-A) zur Herstellung cancerostatischer Mittel, insbesondere zur Generierung einer effektiven Vakzine im tumortherapeutischen Kontext.

## Claims

1. Peptide variants of MUC1 within the VNTR (variable number of tandem repeats) domain entailing 20 amino acids deviating from the known VNTR (variable number of tandem repeats) domain at positions 9, 18 and 19 or at positions 18 and 19 of the PAP20 sequence.

2. Peptide variants according to Claim 1, wherein they manifest the SEQ ID no. 2 PAPGSTAPPAHGVTSAPESR (PAP20-ES).

3. Peptide variants according to Claim 1, wherein they manifest the SEQ ID no. 3 PAPGSTAPAAHGVTSAPESR (PAP20-AES).

4. Immunogenic agent, wherein one peptide according to claims 1 to 3 or the peptide SEQ ID no. 1 PAPGSTAPAAHGVTSAPDTR (PAP20-A) is contained.

5. Antigenic agent, wherein one peptide according to claims 1 to 3 or the peptide SEQ ID no. 1 PAPGSTAPAAHGVTSAPDTR (PAP20-A) is contained.

6. Test kit for the detection of the identity and incidence of DNA mutations within the VNTR domain of the MUC1 containing at least one DNA primer, the sequence of which corresponds to one of the peptides according to claims 1 to 3 or the peptide SEQ ID no. 1 PAPGSTAPAAHGVTSAPDTR (PAP20-A).

7. Use of the peptides according to Claims 1 to 3 or the peptide SEQ ID no. 1 PAPGSTAPAAHGVTSAPDTR (PAP20-A) for the production of cancerostatic agents, in particular for the generation of an effective vaccine in a tumour-therapeutic context.

## Revendications

1. Variantes de peptide du MUC1 du domaine VNTR (variable number of tandem repeats = répétition en tandem en nombre variable) englobant 20 acides aminés qui aux positions 9, 18 et 19 ou aux positions 18 et 19 de la séquences PAP20 divergent du domaine VNTR (variable number of tandem repeats) connu.

2. Variantes de peptide conformément à la revendication 1, se caractérisant par le fait qu'elles présentent la séquence n° d'ident. SEQ ID 2 PAPGSTAPPAHGVTSAPESR (PAP20-ES).

3. Variantes de peptide conformément à la revendication 1, se caractérisant par le fait qu'elles présentent la séquence n° d'ident. SEQ ID 3 PAPGSTAPAAHGVTSAPESR (PAP20-AES).

4. Produit immunogène, se caractérisant par le fait qu'il contient au moins un peptide conformément aux revendications 1 à 3 ou le peptide SEQ ID n° 1 PAPGSTAPAAHGVTSAPDTR (PAP20-A).

5. Produit antigène, se caractérisant par le fait qu'il contient au moins un peptide conformément aux revendications 1 à 3 ou le peptide SEQ ID n° 1 PAPGSTAPAAHGVTSAPDTR (PAP20-A).

6. Kit de test pour démontrer l'identité et l'incidence des mutations d'ADN au sein du domaine VNTR du MUC1 contenant au moins un primaire d'ADN dont la séquence correspond à l'un des peptides conformément aux revendications 1 à 3 ou au peptide SEQ ID n° 1 PAPGSTAPAAHGVTSAPDTR (PAP20-A).

7. Emploi des peptides conformément aux revendications 1 à 3 ou du peptide SEQ ID n° 1 PAPGSTAPAAHGVTSAPDTR (PAP20-A) pour la fabrication de produits cancérostatiques, en particulier pour la génération d'un vaccin efficace dans le contexte de la thérapie des tumeurs.
